# NEW EUROPEAN PATENT SPECIFICATION

(11) **EP 1 139 974 B2**
(45) Date of publication and mention of the opposition decision: **28.11.2018**
(45) Mention of the grant of the patent: 19.10.2011
(21) Application number: 00976694.0
(22) Date of filing: 30.10.2000
(51) Int. Cl.: A61K 8/64, A61Q 19/00

(54) **SOY DEPIGMENTING AND SKIN CARE COMPOSITIONS**
DEPIGMENTIERUNGSMITTEL AUF BASIS VON SOJA ZUR HAUTPFLEGE
COMPOSITIONS DEPIGMENTANTES A BASE DE SOJA POUR SOINS CUTANES

(30) Priority: 05.11.1999 US 163906 P; 03.05.2000 US 201494 P; 27.10.2000 US 698454
(43) Date of publication of application: 10.10.2001
(73) Proprietor: JOHNSON & JOHNSON CONSUMER COMPANIES, INC., Skillman, New Jersey 08558 (US)
(72) Inventor: SEIBERG, Miri, Princeton, NJ 08540 (US); LIU, Jue-Chen, Belle Mead, NJ 08502 (US); SHAPIRO, Stanley, Livingston, NJ 07039 (US); KUNG, John, Somerset, NJ 08873 (US); GROSSMAN, Rachel, Princeton, NJ 08540 (US); MILLER, Jonathan, D., Lawrenceville NJ 08648 (US)
(74) Representative: Kirsch, Susan Edith
(86) International application number: PCT/US2000/029842
(87) International publication number: WO 2001/034099

(56) References cited:
- WO-A1-99/30729
- WO-A2-96/28008
- WO-A2-99/04752

## Description

### Field of the Invention

This invention relates to non denatured soy product-containing compositions that can be used for depigmentation, evening out skin tone and skin texture, skin firming, and care of the skin. More particularly, this invention relates to the use of soybean-containing compositions for depigmentation, producing skin of even tone and color, increasing skin elasticity and reducing skin aging, increasing skin firming and promoting a younger look of the skin, for the prevention and treatment of sun-induced damage, for shine control, for the treatment and prevention of acne, treating cellulite and for inducing aesthetically pleasing skin feel.

### Background of the Invention

The understanding of the chemical and enzymatic basis of melanogenesis is heavily documented. Melanocytes migrate from the embryonal neural crest into the skin to produce secretory granules, melanosomes, which produce melanin. Melanogenesis occurs within the melanosome, and the melanin is later distributed to keratinocytes via the melanocyte dendrites. The key enzyme in melanogenesis is tyrosinase, which initiates a cascade of reactions that convert tyrosine to the biopolimer melanin. Two tyrosinase-related proteins (TRPs) are known, TRP-1 and TRP-2. These proteins share with tyrosinase about 40% homology and have catalytic activities as well as regulatory roles in melanogenesis. TRP-1 is the most abundant glycoprotein in melanocytes.

Skin coloring has been of concern to human beings for many years. In particular, the ability to remove hyperpigmentation, such as found in age spots, freckles or aging skin, is generally of interest to individuals who desire a uniform complexion. In certain areas of the world, general body whitening is desirable. There have been many methods proposed to accomplish depigmentation. For example, kojic acid, hydroquinone, retinoids and other chemical compounds have been used for depigmentation. Many of these previous solutions have not been found acceptable. There is often a distinct line of demarcation between the areas of skin to which such previous compositions have been applied. Therefore, precise application of all these compounds is necessary in order to achieve the desired result. Many of these compounds have also been found to be quite irritating to the skin and therefore undesirable for use.

Post-inflammatory hyper-pigmentation is a frequent problem, representing various cutaneous disorders as well as therapeutic interventions. However, the underlying mechanisms and the variability individuals show for developing post-inflammatory hyper-pigmentation are not well understood. Pulsed laser treatments are not effective for compound, intradermal, or post-inflammatory pigmentation, and are not always affordable. Hydroquinone and broad-spectrum sunscreen are routinely used after therapeutic treatment, but they do not completely prevent or eliminate the hyper-pigmentary lesions. A safe, effective and affordable treatment for post-inflammatory hyper-pigmentation is highly desired. Even more desired is the incorporation of such a treatment into a daily skin care product, which will lead to a healthier and better looking skin.

Aging of the skin is a complex phenomenon resulting from the interaction of several intrinsic and extrinsic factors. Skin changes associated with aging often manifest as cosmetic disabilities. Due to its psychological impact, aging of the skin has become an issue of great social significance and concern. With baby boomers aging, the era of cosmetic care, cosmetic maintenance and rejuvenation gains increased awareness. Methods for preventing and treating skin aging are highly desired. Intrinsic aging is an inevitable, genetically programmed process. Among extrinsic influences (wind, heat, cigarette smoke, chemicals, etc.), ultraviolet radiation appears to be the single most important factor associated with aging of the skin. Photoaging is induced by cumulative exposure to ultraviolet radiation (UVR). Increased recreational sun exposure, including excessive sunbathing, the depletion of stratospheric ozone, and the use of UVR in the treatment of various skin diseases, have led to increased prevalence of photoaging during the last decades. Photodamage can be prevented by sun avoidance and proper sun protection, and could be reversed by the use of topical retinoids, which could be irritating and expensive.
Overexposure to ultraviolet and visible radiation also causes sunburn. The use of aspirin and other nonsteroidal anti-inflammatory drugs, cool baths and topical steroids offer only mild relief.

It is desired to have a single topical treatment that could prevent or reverse skin aging and provide protection or relief from sunburn. It is more desired to have a single and affordable topical treatment for daily skin care, which will provide these benefits, with no irritation or negative side effects, and which will further provide the desired homogeneous skin complexion, even out skin texture and tone, and induce skin firming and a healthier, younger look.

Acne is an inflammatory dermatological disorder which occurs frequently in adolescence and with some regularity in older adults of the human species. The condition of acne can include skin lesions ranging from the comedo in a pilosebaceous follicle, to more severe como-inflammatory symptoms such as pustules, papules, cysts and nodules. The condition is not only uncomfortable for the victim, but also embarrassing, and can result in disfigurement and scarring.

Many different approaches to ameliorating this disorder have been attempted in the past, with some treatments more effective than others. Attacks ranging from simple washing and cleansing to pharmaceuticals have been employed.

It is desired to have a single topical treatment that could prevent or reverse acne, which does not require a pharmaceutical prescription. It is more desired to have a single and affordable topical treatment for daily skin care, which will provide these benefits, with no irritation or negative side effects, and which will further provide the desired homogeneous skin complexion, even out skin texture and tone, and induce skin firming and a healthier look.

Dimpling of the skin of the thighs and buttocks is a phenomenon commonly referred to as cellulite, which affects women much more frequently than men. The basic pathophysiology of cellulite has not been clearly identified. Theoretically, cellulite could reflect differences in adipose tissue biochemistry or connective tissue structure of affected versus unaffected individuals or regions within an individual. There is no evidence of any primary role for adipose tissue physiology, blood flow, or biochemistry in the etiology of cellulite, although the connective tissue of the female thigh is structured to accentuate differences in small sub-dermal adipose tissue depots. Products aimed to cure cellulite (e.g. Cellasene) have been marketed all over the world, but their efficacy is in doubt. Methods to treat cellulite like the solid-probe ultrasound-assisted lipoplasty technique or syringe liposculpture result in some body contouring but are painful and expensive, and needed repeated treatments. It is desired to have a topical affordable treatment that could prevent or reverse cellulite and induce skin firming, resulting in a healthier, younger look of the skin, and provide daily skin care with no irritation or negative side effects.

In our previously filed PCT Patent Application WO 99/04752, the use of non-pasteurized-soybean-derived extracts for the purpose of depigmenting skin was described. We have now found new stabilized and cosmetically superior compositions containing such soy products that contain less than 0.1% surfactants, have greater skin-depigmenting activity and are less expensive to manufacture. We have unexpectedly found that soybean-containing compositions have anti-aging activity, they better increase skin elasticity and firmness, and they are useful in the prevention and in the treatment of sun-induced damage and acne.

### Summary of the Invention

The present invention relates to the cosmetic method of claim 1, the use of claim 2 and the product of claim 3. Preferably, the compositions do not contain more than about 0.1 % of a surfactant. In the use of claim 2 and the product of claim 3 the composition may further comprise one or more other skin-related active agents such as, but not limited to, depigmenting agents, thickening agents, emollients, antioxidants or sunscreens.

The present invention also describes a method to enhance the care of the skin. We unexpectedly found that application of soymilk compositions increase skin firmness and elasticity, even tone and texture, reduce skin aging, and are useful in the prevention and in the treatment of sun-induced damage and acne.

Other features and advantages of the present invention will be apparent from the detailed description of the invention and from the claims

### Brief Description of the Figures

Figure 1 shows three graphs, demonstrating that soymilk can inhibit trypsin, inhibit PAR-2 cleavage, and inhibit keratinocyte phagocytosis.
Figure 2 shows histological sections of swine skin treated with soy formulations and stained to demonstrate pigment deposition.
Figure 3 shows a picture of a flank of a dark skinned swine, treated with soymilk and DHLA formulations.
Figure 4 shows histological sections of mice skins treated with soymilk and stained to document elastin fibers.
Figure 5 shows pictures of an individual excessively exposed to the sun, following treatment of a half of his face with soymilk.
Figure 6 shows a magnification of the skin of an individual excessively exposed to the sun, following treatment of a half of his face with soymilk.
Figure 7 shows the results of an acnegenicity and irritancy study in graphical form.
Figure 8 shows a Western blot of soy formulations probed for STI (Soybean Trypsin Inhibitor).

### Detailed Description of the Preferred Embodiments

It is believed that one skilled in the art can, based upon the description herein, utilize the present invention to its fullest extent. The following specific embodiments are to be construed as merely illustrative and not limitative of the remainder of the disclosure in any way whatsoever. Unless defined otherwise, all technical and scientific terms used herein have the same meaning as commonly understood by one of ordinary skill in the art to which the invention belongs. Also, all publications, patent applications, patents, and other references mentioned herein are incorporated herein by reference.

The novel compositions of this invention contain non-denatured soy milk. What is meant by "soy product" is a substance derived from the soybean, containing the ingredients naturally found in soybeans, at the relative concentrations as found in the beans. The soy product is a non-denatured soy product. "Denaturation" is defined in the Bantam Medical Dictionary (1990 edition) as "the change in the physical and the physiological properties of a prottein, that are brought about by heat, X-rays or chemicals. These changes incllude loss of activity (in the case of enzymes) and loss (or alteration) of antigenicity (in the case of antigens)". What Is meant by "non-denatured soy product" is a soy product in which the processing for the derivation of such soy product (e.g., the temperature, extraction media) did not eliminate its protease inhibitory activity. In one embodiment, the non-denatured state of the soy product of this invention Is measured by the presence of an Intact soybean trypsin inhibitor (STI) protein.

The soy product is soymilk. One way to make soymilk is to soak the soybeans in deionized or purified water for several hours, and grind them after they were fully hydrated, with the addition of small quantities of water. (The grinding process allows the soybean milk to be extracted). After collection, the soybean milk may be filtered to remove any residual parts of the bean husk. The soymilk used in this invention can be fresh soymilk as described above, or may be made from soybean powder and water. The soybean powder is milled from soybeans and may also be lyophilized, spray dried, or freeze-dried and the resulting soymilk may or may not be filtered. Such prepared soymilk may have from about 1 to about 90% by weight dry soybean powder. Another example is the use of soymilk powder, made from lyophilized, spray dried or freeze-dried soymilk, with the addition of water and finished with or without filtration or homogenization.

Other methods of soybean extraction could also be used to create the active ingredients used in this invention. For example, but not limited to, the active ingredients could be extracted from ground soybeans using ethanol/water mixtures, followed by the removal of the ethanol from the extract, in such ways that the protease inhibitory activity of the soybean will be retained.

The compositions of the present invention may contain from about 1% to about 99%, by weight, of the soy product. The composition may contain from about 50% to about 99%, by weight, (e.g., from about 70% to about 99%) of the liquid soy product. For example, when a solid soy product (e.g., soybean powder or soymilk powder) is used, the composition may contain from about 1% to about 50%, by weight (e.g., from about 2% to about 30%, by weight) of the solid soy product. Composition which comprises solid soy products may also comprise water (e.g., distilled water or water contained within soymilk) to form a liquid base to the composition (e.g., to form a cream, lotion, injectable solution or gel). Such composition may comprises from about 50% to about 98%, by weight (e.g., from about 70% to about 98%, by weight) of water. While not limited to these methods of administration, the compositions of this invention may be delivered topically, orally, or parenterally.

The soy products useful in this invention may be produced from all soybean species, regardless of their geographic origin, sun exposure, harvest time and the like. However, specific strains, geographic origins or growth conditions might be preferred. For example, but not limiting to, soybean strains or other legume strains particularly rich in their trypsin inhibitor (e.g. STI, LTI, BBI) content or growth conditions that result in trypsin inhibitor enrichment in the bean, might be preferred. It should be noted that the legume products useful in the compositions of this invention have a distinctive odor, which may be tolerable in some cultures, but is undesired in others. If necessary, the odor of the compositions of this invention may be reduced by using soybean products derived from specific strains of soybeans known to produce reduced-odor, including, but not limited to, lipoxygenase-2-deficient beans and those having modified sugar profile, and the like. A process to reduce oxygen levels in the formulation may also reduce the odor. Various masking agents or fragrances may also be used to mask the odor.

The skin composition used in the cosmetic method of claim 1 contains a preservative. In the composition used in claim 2 and the use of claim 3, the one or more preservative is optional. Due to the fact that the compositions of this invention are non-denatured (e.g., compositions in which the protease inhibitory activity is retained), they may be more favorable as a medium for microbial growth. Preservatives are useful for substantially preventing microbial decomposition. Examples of preservatives include phenoxyethanol and parabens such as methyl-paraben, ethyl-paraben, and propyl-paraben. Other examples of preservatives are listed on pages 1654-55 of the International Cosmetic Ingredient Dictionary and Handbook, eds. Wenninger and McEwen (CTFA, 7th ed., 1997), hereinafter referred to as the "Cosmetic Handbook." The composition may comprise from about 0.01% to about 20%, by weight (more preferably, from about 0.5% to about 5%, by weight) of preservative. Microbial contamination can also be eliminated by gamma irradiation or microfiltration, or by brief heat treatments that do not result in the elimination of protease inhibitory activity.

The skin composition used in the cosmetic method of claim 1 also contains an antioxidant and a chelating agent. For the use of claim 2 and the product of claim 3 antioxidants and/or chelating agents may also be used to increase shelf life and stability of the compositions. Antioxidants may be added both for formulation stabilization and for biological efficacy. Antioxidant compounds and their derivatives include, but are not limited to, water-soluble antioxidants such as sulfhydryl compounds and their derivatives (e.g., sodium metabisulfite and N-acetyl-cystein), lipoic acid and dihydrolipoic acid, resveratrol, acetyl-cysteine (Iniferine®) or lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, propolis, and legume extracts. Other examples of antioxidants may be found on pages 1612-13 of the Cosmetic Handbook. The compositions of the present invention may comprises the antioxidant in an amount of from about 0.001% to about 20%, by weight (e.g., from about 0.01% to about 10% by weight) of the composition.

Chelating agents are also useful in assisting the stabilization of the compositions of this invention. Examples of chelating agents include EDTA and derivatives thereof (e.g., disodium EDTA and dipotassium EDTA), Iniferine ®, lactoferrin, and citric acid. Other examples of chelating agents are listed on page 1626 of the Cosmetic Handbook. The compositions of the present invention may comprise the chelating agent in an amount of from about 0.001% to about 20%, by weight (e.g., from about 0.01% to about 10% by weight) of the composition.

Thickening agents are present in the composition used in the cosmetic method of claim 1. In the composition used in claim 2 and the use of claim 3, the thickening agent is optional. Thickening agents (e.g., thickeners or viscosity enhancing agents) may be utilized in the compositions of this invention to alter their viscosity. The desired viscosity of the composition will depend upon the intended use (e.g., as a bath product, cream, lotion, or gel). For example, in applications such as bath or wash products, the viscosity of the composition should be relatively low, similar to an aqueous solution. Application as a cream, lotion, or gel will have slightly higher viscosity (e.g., between about 100 cps and 100,000 cps).

Thickening agents that can be added to the compositions of this invention to alter viscosity include polymers such as polyacrylates (e.g., polyacrylamide). Other examples of viscosity modifying agents are listed on pages 1692-97 of the Cosmetic Handbook. To achieve the appropriate viscosity, compositions of the present invention may comprise from about 0.01% to about 20%, by weight (e.g., from about 0.1% to about 5%, by weight) of a thickening agent.

The compositions used in claim 2 and the product of claim 3 may also contain other depigmenting agents in addition to the soy product. Examples of such depigmenting agents include, but are not limited to, lipoic acid, dihydrolipoic acid, resveratrol, ascorbic acid, kojic acid, hydroquinone, isoflavones, retinoids (e.g., retinol, retinoic acid, and retinyl palmitate), tyrosinase inhibitors, melanosome transfer inhibitors, and selective cytotoxic agents for melanocytes, or natural extracts containing these activities. The amount of the depigmenting agent used will depend on the activity of the compound, and will typically range from about 0.001% to about 20%, by weight (e.g., from about 0.01% to about 10%, by weight) of the composition.

The composition of the present invention may also contain compounds that enhance the feel of the composition on the skin of the user. Examples of such compounds include, but are not limited to, oils, silicones (e.g.,-siloxane polymers such as dimethicone) and skin-conditioning agents such as emollients, and humectants. Examples of such skin conditioning agents may be found of pages 1656-1670 of the Cosmetic Handbook.

The compositions used in claim 2 and the product of claim 3 may also advantageously contain other cellulite-combating agents including, but not limited to, caffeine, retinol and other retinoids and the like. Of course, other topical agents that may have activity on the skin, including anti-inflammatory agents such as corticosteroids, anti-pruritic agents, topical analgesics, agents which fight acne, such as benzoyl peroxide and beta-hydroxy acids such as salicylic acid, anti-aging products including retinoids, retinol, alpha hydroxy acids, co-enzyme-Q, and others, antibiotics and antimycotics and shine-control products including miconazole, ketoconazole, elubiol, itraconazole and the like, and others known to those of ordinary skill in the art, may be combined in the compositions used in claim 2 and the product of claim 3.

Compositions of the present invention may be prepared by mixing the desired ingredients. For example, soymilk is mixed with the chelating agent, preservative, and/or antioxidant. A thickener is then added to the system, and the mixture is further mixed until it reaches homogeneity at the desired viscosity. The compositions of the present invention may be prepared under an argon or nitrogen (or other inert gaseous) blanket in order to enhance formulation stability and/or to reduce soybean odor. The compositions of this invention may be packaged in a tube, a sealed packet, a jar, a pump, a bottle, a can, a pledget, a towelet, a wipe or the like. An airtight package such as an aluminum tube, aluminum pocket, pump, laminate tube, can also be used to further enhance product stability.

The compositions of the present invention may be used to depigment the skin of a mammal (e.g., a human). What is meant by depigmentation is the lightening of the color of an area of skin, including but not limited to, the global lightening of the user's skin tone/complexion (e.g., the face, hands, or whole body, which is uneven as a result of aging skin, or darker than desired because of ethnicity or pathology, and the like), the evening of skin color tone, or the specific lightening of age spots, freckles, or darker pigmented areas such as, but not limited to, post-inflammatory hyper-pigmentary lesions.

We unexpectedly found that the compositions of this invention may be used to treat human skin resulting in reduced skin oiliness and shine. The compositions of this invention, including, but not limited to soy-containing compositions, which contain protease inhibitory activity, when applied topically to the skin, including its use as a bath additive, or in a wipe, reduced the oily and shiny look of facial skin. In one prefered embodiment, the composition may be administered at least once daily for as long as the user wants the desired effect.

Compositions of the present invention may also be used to prevent or treat the condition of acne. The compositions of this invention may be applied, preferably, twice per day for a period of time preferably at least about 45 days to demonstrate an improvement in the appearance of the skin and an amelioration of the condition. However, it is thought that application of the compositions for at least about two to four weeks should provide an amelioration or prevention of the condition, depending on the individual's sebaceous gland activity.

In another embodiment, we found that soy products, such as but not limiting to the compositions of this invention, which contain protease inhibitory activity, when applied topically to the skin, including its use as a bath additive, or in a patch, assists in creating a soothing, cooling, and/or relaxing effect for the user, and an aesthetically pleasing skin feel. The amount of composition administered and the frequency of administration will depend upon the concentration of the soy agent in the composition (e.g., the soymilk), the condition of the skin area being treated, and the desired effect and, thus, will ultimately be determined by the user of the composition. In one embodiment, the composition is administered at least once daily for as long as the user wants the desired effect.

Moreover, we unexpectedly found that soymilk treatment of human skin following an excessive exposure to the sun, relieved the pain and burning feelings and reduced the skin scaliness associated with sunburn. This invention provides a method for the soothing, cooling and pain reducing effects following sunburn or excessive sun exposure. The method consists of daily topical treatment at least once per day for as long as the user wants the desired effect, of soymilk containing compositions that contain protease inhibitory activity.

In another embodiment we unexpectedly found that pre-treatment of human skin with soymilk (e.g., topical treatment, use as bath additive) could protect from sun-induced damage. Skin over-exposed to the sun following soymilk treatment was not as red, scaly, dry and painful, as the untreated skin of the same individual. This invention provides a method for the prevention of skin scaling, redness and pain following excessive sun exposure. The method consists of daily topical treatment at least once per day for as long as the user wants the desired effect, of soymilk containing compositions that contain protease inhibitory activity.

Thus, soymilk and its derivatives can provide skin care properties, including elasticity, prevent scaliness/redness/pain of over exposure to the sun, after sun/sunburn, cooling sensation, bath, depigmentation, even tone and texture of the skin, post inflammatory hyperpigmentation, acne and cellulite.

As evidenced by the examples herein, we have unexpectedly found that the topical use of soymilk results in an increase in the elastic properties of the treated skin. Therefore, suitable topical use for legume extracts containing protease inhibitory activity, and in particular for soymilk and soybean formulations, is to increase skin elasticity and firming, by affecting Elastin and collagen fibers and skin elastic properties. We unexpectedly found that the topical application of soy products, including but not limiting to the compositions of this invention and of our previous invention (PCT Patent Application WO 99/04752), which contain protease inhibitory activity, results in skin firming, increased skin elasticity, and anti-aging effects, and induced an aesthetically pleasing skin feel. This invention provides a method for increasing skin firming and skin elasticity, and reducing signs of skin aging, which is useful for all body parts, and preferably for the face, upper body, and of the skin of the thighs and buttocks. The method consists of daily topical treatment at least once daily for as long as the user wants the desired effect, of soymilk containing compositions that contain protease inhibitory activity.

The following examples illustrate several embodiments of the compositions of this invention. They should not be utilized to limit or narrow the scope of the invention in any way.

### Reference Example 1: Preparation of Soymilk from Soybean Powder

160 g of soybean powder (Sunlight Foods, Taipei, Taiwan) was added to about 1440 g of deionized water. The mixture was stirred at room temperature for about 1 hour. The mixture was then filtered through a sieve having holes of 75µm diameter. The filtrate resulted in about 1.1 kg of soymilk.

### Reference Example 2: Preparation of Soymllk Gel from Soymilk

The following compositions were prepared as follows. The weight percentages of each ingredient in the compositions are indicated below in Table 1 and Table 2. First, the soymilk, as prepared in example 1, was placed into a first beaker. The preservative Phenonip® (a mixture of the preservatives methyl-paraben, propyl-paraben, ethyl-paraben, and phenoxy-ethanol sold by NIPA, Wilmington, Delaware) or the preservative phenoxyethanol were added to the soymilk. Next, the chelating agent Disodium EDTA and in some examples the humectant glycerin were added to the first beaker and mixed with the soymilk. It is also possible to further add cyclomethicone, or dimethicone (tradename Dow Corning 200 Fluid ®), or PolySorbate 20, or Aluminum Starch Octyl Succinate, or Sucrose Cocoate, or PEG-6 Capric/Caprylic Triglycerides to the soymilk mixture at this step as required in some examples in Table 1 and Table 2. A mixture of the thickener polyacrylamide, laureth-7, and C13-14 isoparaffins (sold by Seppic, Paris, France under the Tradename Sepigel®) was added to a second beaker along with the anti-oxidant BHT. The ingredients in the second beaker were then added to the ingredients of the first beaker and mixed until homogenous. The anti-oxidants ascorbic acid, sodium ascorbyl phosphate, lactoferrin, or tocopherol were then added to the beaker and homogeneously mixed to form the resulting gel.

### Reference Example 3: Preparation of Soymilk Gel from Soybean powder, Soymilk Powder or Soybean Extract

The following compositions were prepared as follows. The weight percentage of each ingredient in each of the preparations is indicated below in Table 2. First, the soymilk powder (Devansoy Farms, Carroll, IA) or the soybean powder (Sunlight Foods, Taipei, Taiwan) or the Soybean Extract and deionized water were placed into a first beaker and mixed to reconstitute the soy powder. The preservative Phenonip® and the chelating agent Disodium EDTA were then added to the first beaker and mixed with the soymilk. A mixture of polyacrylamide, laureth-7, and C13-14 isoparaffins was added to a second beaker along with the anti-oxidant BHT. The Ingredients in the second beaker were then added to the ingredients of the first beaker and mixed until homogenous.

**Table 1**

| **Soybean Essences** | | | | | |
|---|---|---|---|---|---|
| | **1** | **6** | **8** | **21** | **23** |
| Soymilk | 87.42% | 89.04% | 96.09% | 96.05% | 95.70% |
| Phenoxyethanol | 0.73% | | | | |
| Phenoxyethanol and Parabens | | 1.00% | 1.00% | 1.00% | 1.00% |
| Glycerin | 2.50% | 2.50% | | | |
| Cyclomethicone Aluminum | 2.00% | | | | |
| Starch Ocetyl Succinate | 0.75% | | | | |
| Sucrose Cocoate | 1.00% | 1.00% | | | |
| PEG-6 Capric/Caprylic Triglycerides | 3.00% | 3.00% | | | |
| Disodium EDTA | 0.10% | 0.10% | | | 0.05% |
| Polyacrylamide/Laureth-7/C₁₃₋₁₄ Isoparrafin | 2.50% | 2.75% | 2.90% | 2.90% | 3.20% |
| Ascorbic Acid | | 0.01% | | | |
| Butvlated Hydroxytoluene | | 0.10% | 0.01% | 0.05% | 0.05% |
| Polysorbate 20 | | 0.50% | | | |
| TOTAL | 100% | 100% | 100% | 100% | 100% |

**Table 2**

| | **24** | **26** | **27** | **28** | **33** | **34** | **35** |
|---|---|---|---|---|---|---|---|
| Sovmilk | 94.40% | 92.40% | 90.70% | 94.70% | | | |
| Phenoxyethanol and Parabens | 1.00% | 1.00% | 1.00% | 1.00% | 1.00% | 1.00% | 1.00% |
| Glycerin | | | 5.00% | | | | |
| Disodium EDTA | 0.05% | 0.05% | 0.05% | 0.05% | 0.05% | 0.05% | 0.05% |
| Polyacrylamide/Laureth-7/C₁₃₋₁₄ Isoparrafin | 3.50% | 3.50% | 3.20% | 3.20% | 3.20% | 3.20% | 3.20% |
| Ascorbic Acid | | 1.00% | | | | | |
| Butylated Hydroxytoluene | 0.05% | 0.05% | 0.05% | 0.05% | 0.05% | 0.05% | 0.05% |
| Deionized Water | | | | | 90.70% | 90.70% | 85.70% |
| Lactoferrin | 1.00% | 1.00% | | | | | |
| Tocopherol | | 1.00% | | | | | |
| Dow Corning 200 Fluid | | | | 1.00% | | | |
| Soymilk Powder | | | | | 5.00% | | |
| Soybean Extract using Ethanol/Water Mixture | | | | | | 5% | 10% |
| TOTAL | 100% | 100% | 100% | 100% | 100% | 100% | 100% |

### Reference Example 4: Soymilk inhibits the PAR-2 Pathway

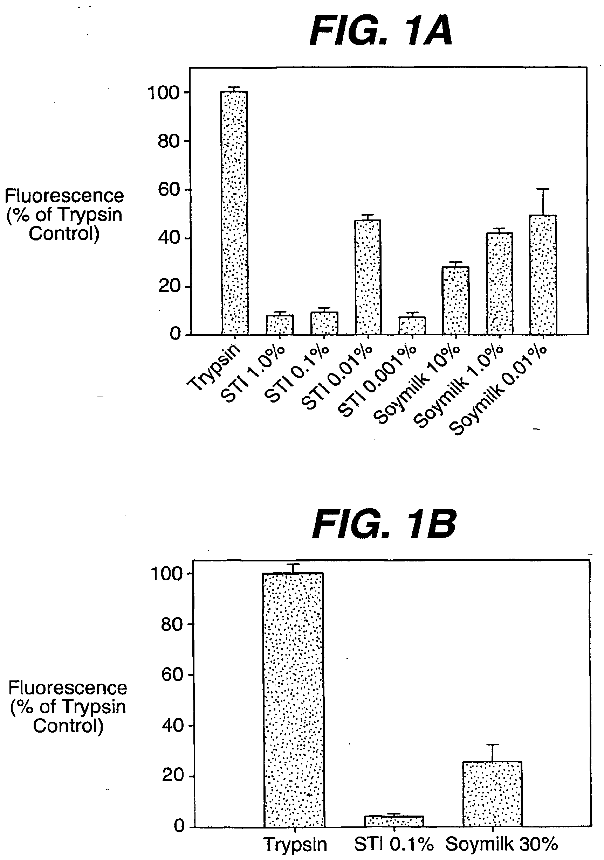

Our patent application WO 99/04752 describes the importance of the PAR-2 pathway in pigmentation, and shows that the soybean-derived protease inhibitor STI inhibits this pathway and induces depigmentation. Our patent application WO 99/30729 further describes the role of PAR-2 in inducing keratinocyte phagocytosis and its effect on melanosome transfer and pigmentation. To test the possibility that soymilk induces depigmentation by inhibiting PAR-2 induced phagocytosis the following experiments were performed. First, STI and soymilk were shown to act as serine protease inhibitors, by inhibiting the trypsin-induced cleavage of a fluorescent casein peptide in a dose-dependent manner (Figure 1a). Total protease activity was measured using the EnzChek™ protease assay kit, following manufacturer's instructions (Molecular Probes). STI and soymilk were diluted in PBS (Life Technologies) and incubated at 1:1 (v/v) with 1000 units of trypsin, prepared in PBS. Following incubation with BODIPY fluorescent casein substrate at room temperature for one hour, fluorescence was measured (excitation 505/emission 515) on a SpectraMax^{®} Gemini microtiter plate reader (Molecular Devices Corporation) using Softmax^{®} Pro 3.0 software (Molecular Devices Corporation). Each experiment was performed in six replicates and was repeated three times. The percent inhibition of trypsin cleavage of the substrate by STI and soymilk was calculated using Microsoft Excel and graphed in Sigma Plot. As shown in Figure la, soymilk can inhibit trypsin-induced cleavage in a dose response manner.

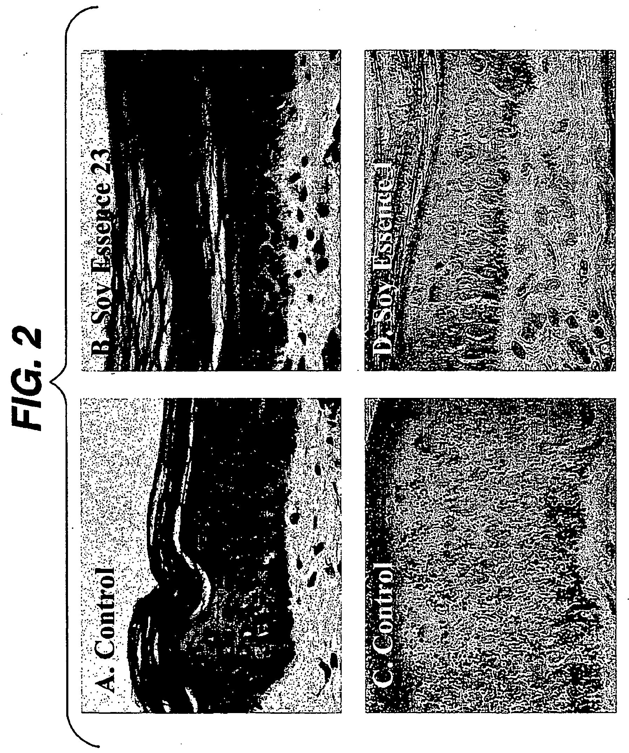

In a second experiment, a similar assay was designed to test the possible specific inhibition of soymilk on PAR-2 cleavage. In this assay, a synthetic fluorescent peptide comprising the cleavage site of the human PAR-2, SKGRSLIGK (a similar assay is described in: Lourbakos A, Chinni C, Thompson P, Potempa J, Travis J, Mackie EJ, Pike RN, Cleavage and activation of proteinase-activated receptor-2 on human neutrophils by gingipain-R from Porphyromonas gingivalis., FEBS Lett 435: 1, 45-8, Sep 11, 1998), replaced the casein peptide as a substrate for trypsin. The peptide was labeled with the fluorophore pair Edans/Dabsyl (Advanced Bioconcept, Montreal, Canada), and was used as a substrate for PAR-2 serine protease activators. Cleavage of this peptide with PAR-2 activators such as trypsin could be detected fluorescently (excitation 335 / emission 515). The inhibition of trypsin-induced PAR-2 peptide cleavage by STI or Soymilk was measured by incubating 100 micromolar peptide with10 units of trypsin (in 100mM TRIS [hydroxymethyl]aminomethane buffer, pH 8.0, Digene, Beltsville, MD), with or without the test material, for one hour at room temperature, protected from light. Fluorescence was measured on SpectraMax^{®} Gemini microtiter plate reader (Molecular Devices Corporation) using Softmax^{®} Pro 3.0 software (Molecular Devices Corporation). Each experiment was performed in six replicates and was repeated three times. The percent inhibition of trypsin cleavage was calculated using Microsoft Excel and graphed in Sigma Plot. As shown in Figure 1b, the cleavage of this PAR-2 peptide by trypsin was completely inhibited in the presence of STI. Soymilk retained the ability to inhibit this trypsin-induced PAR-2 cleavage (Figure 2b), suggest that similar to STI, soybean extracts could inhibit PAR-2 activation.

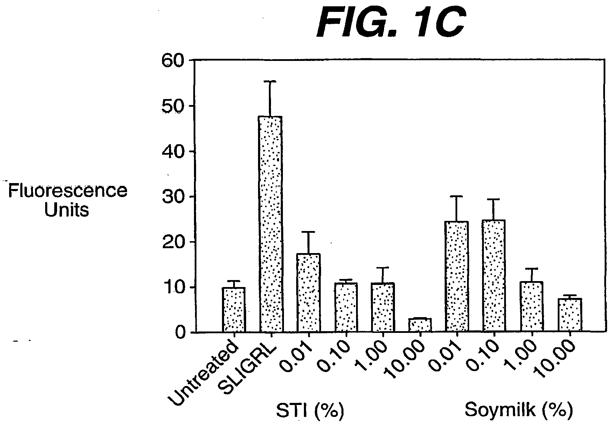

To examine the possibility that soymilk could inhibit PAR-2 induced keratinocyte phagocytosis, we used the Vybrant™ Phagocytosis Assay (Molecular Probes). HaCaT keratinocytes were treated with SLIGRL, the PAR-2 activating peptide, and increasing concentrations of STI or soymilk for 24 hours, following by incubation with 100 microliter of fluorescein-labeled *E. coli* K-12 bioparticles for four hours. Intracellular fluorescence, indicative of ingested *E. coli par*ticles, was measured after trypan blue quenching of the non-internalized fluorescence. Fluorescence was measured (485 nm excitation/538nm emission) using a SpectraMax^{®} Gemini microtiter plate reader (Molecular Devices Corporation, Sunnyvale, CA). Data was collected using Softmax^{®} Pro 3.0 software (Molecular Devices Corporation), and was analyzed using SigmaPlot^{®} 5.0 (SPSS Science, Chicago, IL) and SigmaStal^{®} 2.0 (SPSS Science) software. Each experiment was performed in six replicates and was repeated at least three times. As shown in Figure 1c, SLIGRL increased keratinocyte phagocytosis, and STI prevented this increase in a dose dependent manner. Interestingly, freshly prepared soymilk was able to inhibit SLIGRL induced phagocytosis in keratinocytes to a similar degree. These three experiments indicate that soymilk contains a protease inhibitory activity that inhibits PAR-2 activation, which leads to reduced keratinocyte phagocytosis. This data suggest that the depigmenting activity of the soy products is due, in part, to their PAR-2 inhibitory activity.

### Example 5: The depigmenting activity of new soymilk formulations is superior

Our patent application WO 99/04752 documents the depigmenting activity of soymilk, and presents soymilk-containing formulations for the use of depigmentation. An example of such formulation is Soy Essence 1, as described in Table 1 above. The present invention describes depigmenting compositions comprising a soy product and a stabilizing system but with no more than 0.1% surfactant. An example of such formulation is Soy Essence 23, as described in Table 1 above. To demonstrate that Soy Essence 23 is superior to Essence 1 in its depigmenting activity, the following experiment was performed.

Dark skinned Yucatan microswine (Charles River, Maine) were housed in appropriately sized cages in an environmentally controlled room with a 12-hour light - 12-hour dark photoperiod and supplied with food and water ad *libitum*. Animal care was based on the "Guide for the Care and Use of Laboratory Animals", NIH Publication No. 85-23. Twenty microliter of test compounds were applied topically, twice a day, five days/week, for eight or nine weeks, on the dorsum of the swine. Treatments of individual swine were always arranged in a head to tail order on one side, and in a tail to head order on the other side of the animal. Biopsies were taken using standard techniques. All swine studies showed no visual irritation, and histological analyses revealed no markers of irritation or other pathological signs.

Sections from biopsies were stained with Fontana-Mason (F&M), using standard procedures (Sheenan and Hrapckak, 1980). F&M staining identifies silver nitrate reducing molecules. In skin, this non-specific stain identifies primarily melanin. At least three sections per biopsy were processed. Each experiment was repeated at least three times.

Figure 2 shows histological sections from two such experiments. The sections were stained with F&M, to mark the melanin deposition in the treated sites. The top panel shows the control (a) and the Soy Essence23 (b) treated sites of one swine. The lower panel shows the control (c) and the Soy Essence1 (d) treated sites of another swine. It is obvious from this figure that the both the relative and the absolute reduction in pigment deposition induced by Soy Essence 23 is superior to the level induced by Soy Essence1.

### Example 6: The depigmenting activity of soymilk is enhanced when combined with antioxidants

Our patent application WO 99/04752 documents the depigmenting activity of soymilk. As we show here, this depigmenting activity is due, in part, to the inhibition of PAR-2 induced keratinocyte phagocytosis. To test the hypothesis that improved depigmentation could be achieved by combining more than one mechanism that lead to depigmentation, we tested the depigmenting effect of soymilk and anti-oxidant combinations.

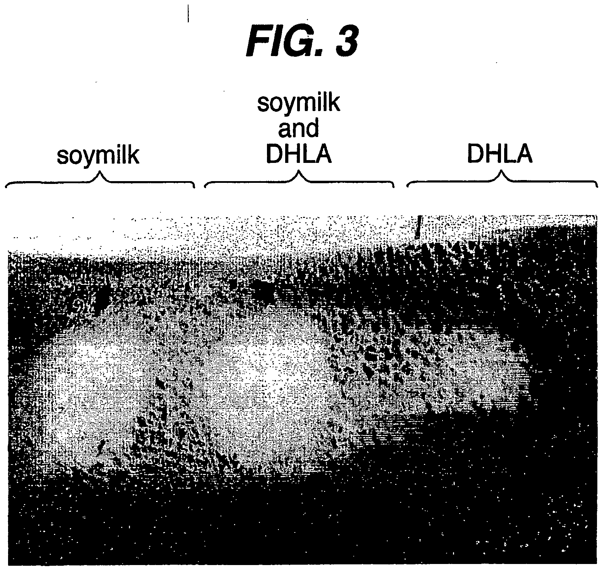

Swine were treated with soymilk or Soymilk Essence formulations, 1% dihydrolipoic acid (DHLA), and 1% Resveratrol (Res), twice daily, or with combination treatments of soymilk once daily and DHLA or Res once a day. Visual skin lightening was observed after seven or eight weeks of treatment. An example of such experiment is demonstrated in Figure 3, showing a flank of a dark skinned swine following eight weeks treatment with soymilk Essence and DHLA combination. The left site(1) is treated with Soymilk Essence23, a soymilk formulation described above. The right site (3) is treated with DHLA, and the middle site (2) is treated with both Soymilk Essence 23 and DHLA. This figure clearly demonstrates increased depigmentation following the combination treatment of Soymilk essence 23 and DHLA, relative to treatments with each ingredient alone. Similar results were obtained using soymilk or soymilk essence, combined with DHLA or Res. This example clearly demonstrates that additives of different mechanism can improve the depigmenting effect of soymilk.

### Example 7 : Soymilk affects skin elasticity.

While performing the soymilk studies described in our applications JBP-430, JBP-464 and JBP-467, we unexpectedly noticed that the skin of soymilk treated mice, upon palpation, felt more elastic. To test the possibility that soymilk has a positive effect on skin elasticity, we performed two sets of experiments: non-invasive elasticity measurements (cutometer measurements) and staining of skin section for Elastin fibers. These studies indicate that soymilk treatment increases Elastin fibers in skin, and the treated skin has increased elastic properties.

C3H and C57B1/6 mice were either induced for a new hair cycle as described in our applications JBP-430, JBP-464 and JBP-467, or shaved. Mice were treated with soymilk daily as described in the above applications. At the start of the experiment (day 1, before first treatment), and at day 21, a cutometer measurement was performed to measure skin elasticity. We used a cutometer available from Acaderm (Menlo Park, California), and employed the methods described in Couturaud et al., "Skin Biomechanical Properties: In Vivo Evaluation of Influence of Age and Body Site by a Non-Invasive Method," 1 Skin Res. and Technol. 68-73 (1995) and Elsner et al., "Mechanical Properties of Human Forearm and Vulvar Skin," 122 Br. J. Dermatol. 607-614 (1990) which are both incorporated herein by reference in their entirety. Suction was applied through a 2 mm aperture and the corresponding skin displacement and recovery after release of the negative pressure were measured. In human studies, an improvement in the ratios of deformation parameters Ua/Uf (skin fatigue, or total recovery from the load), Ur/Uf (biological elasticity, or elastic recovery after loading), and Ur/Ue (firmness, or improvement in the deformation resistance of the skin) indicates better tonicity and elasticity of the skin. The deformation parameters Ue, Uf, Ua, and Ur are dependent, in part, on skin thickness. Consequently, ratios were used for evaluation as described in Barel et al., "Suction Method for Measurement of Skin Mechanical Properties: The Cutometer," Handbook of Non-Invasive Methods and the Skin 335-340 (1995) which is incorporated herein by reference in its entirety.

An example of one such experiment is shown in Table 3 demonstrating that soymilk treatment increases these parameters, which reflect improved skin elasticity. While variations between animals were significant, the increase in cutometric properties was consistent, reflecting the beneficial effect of soymilk to skin elasticity.

**Table 3: Mechanical Properties of shaved, soymilk-treated C3H mice skin**

| Biophysical Parameter | Day 1 | | Day 21 | |
|---|---|---|---|---|
| | Untreated Control | Soymilk Treated | Untreated Control | Soymilk Treated |
| Ua/Uf | 0.860 +/- 0.066 | 0.815 +/- 0.154 | 0.839 +/- 0.068 | 0.904 +/-0.043 |
| Ur/Ue | 0.943 +/- 0.172 | 0.964 +/- 0.103 | 0.617 +/- 0.151 | 1.402 +/-0.846 |
| Ur/Uf | 0.720 +/- 0.115 | 0.677 +/- 0.168 | 0.506 +/- 0.136 | 0.787 +/-0.067 |

To further study this elasticity effect, skin biopsies were taken throughout this experiment, and sections were stained for Elastin in accordance with the methods set forth in Kligman, L.H., "Luna's Technique, A Beautiful Stain for Elastin," 3(2) The Amer. J. of Dermatopathol. 199-200 (1981) which is incorporated herein by reference in its entirety.

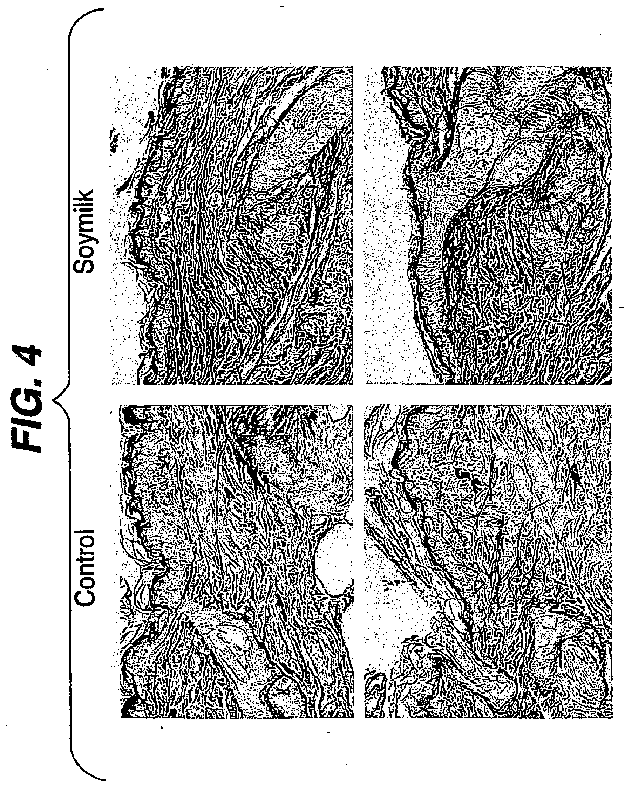

As shown in Figure 4, Elastin fibers (stained dark blue-purple) were increased in thickness and density around the skin appendages and the upper dermis of the soymilk- treated C3H mice (right panel) when compared to the untreated controls (left panel). This increase was detectable as early as at the forth day of treatment, and was consistent throughout the study. Same results were also obtained using the C57B1/6 mice. This example demonstrates that the use of soymilk and soymilk formulations for skin care would increase skin elasticity and reduce skin aging.

In a separate experiment, the effect of soy powder on collagen synthesis was studied *in vitro.* The effect of soy powder to enhance the rate of collagen synthesis in normal human dermal fibroblasts was assessed by evaluating the incorporation of radioactive proline in extracellular collagen after three days of treatment. As shown in Table 4, soy powder enhanced the rate of collagen synthesis in normal human dermal fibroblasts at 1 µg/ml by 58%. At lower concentrations, no significant stimulation was seen with soy powder. Thus, the skin appeared more firm, full and more youthful.

**Table 4: Effect of Soy powder on extra-cellular collagen synthesis in normal human dermal fibroblasts.**

| | | Soja (µg/ml) | | |
|---|---|---|---|---|
| | Control | 0.01 | 0.1 | 1 |
| Collagen synthesis (dpm/cell)*1000 | 11.26 ±1.62 | 15.0 ±1.46 | 13.68 ±2.51 | 17.80 ± 2.01 |
| | | NS | NS | p < 0.05 |
| Stimulation | | +33% | +21% | +58% |
| NS: non significant. | | | | |

### Example 8: Soymilk prevents sun-induced damage.

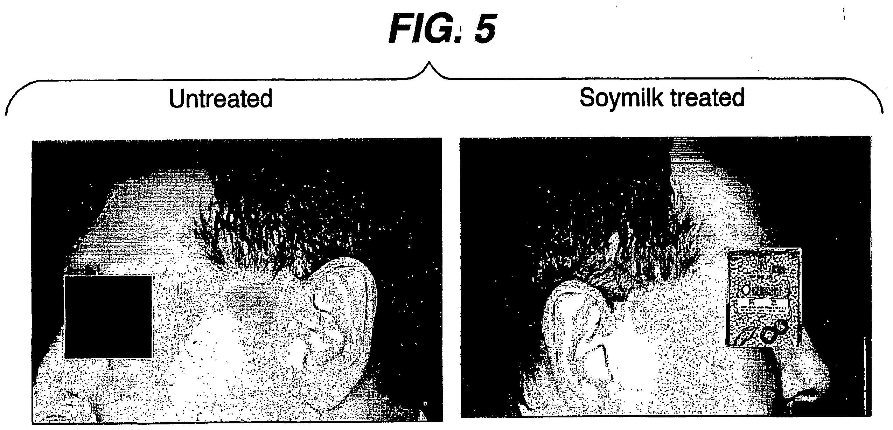

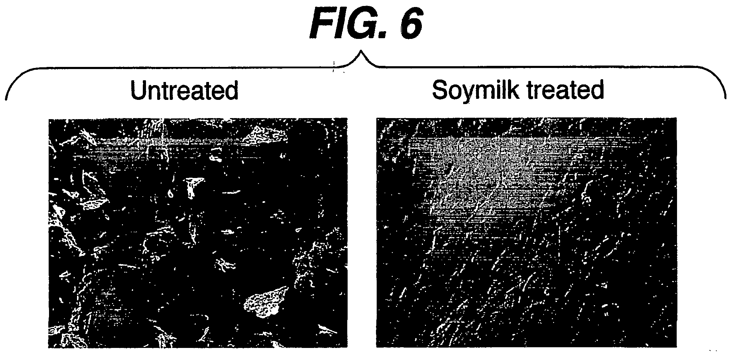

One human individual was treated with a composition according to this invention containing soymilk on the right half of his face, once daily, immediately after shaving. After eight weeks of soymilk treatment, this individual was excessively exposed to the sun, during recreational activity, without the use of a sunscreen. The individual's skin exhibited the symptoms of "sun-burn": his face turned red and painful, and scaliness was developed by the second and third day after sun exposure. Unexpectedly, the right side of his face, having been pre-treated with a soymilk composition according to this invention, was not red, painful or scaly. Figure 5 shows the two sides of the individual's face. The differences in skin redness and in the degree of sun damage are obvious from this figure. Figure 6 shows Hi-scope images of the individual's skin, demonstrating the scaliness of the untreated side and the smoothness of the treated side. This example demonstrate that the routine use of soymilk compositions according to this invention, could protect the skin from unexpected strong sun exposure, and reduce redness, scaliness and pain associated with "sun burns".

### Example 9: Soymilk can treat sun-induced damage.

The individual described in Example 7 also used a soymilk composition on a part on his untreated side, only at 24 and 48 hours post sun-exposure. The application of soymilk after sun exposure served not only to moisturize the damaged skin. It was found to induce cooling and soothing sensation, to relieve the pain associated with the excessive sun exposure, and to reduce redness and scaliness while leaving a "fresh" feeling. This example demonstrates that topical application of soymilk or soymilk formulations could relieve the pain and reduce the redness and scaliness resulted from excessive sun exposure. The use of soymilk on sun-exposed skin leads to a cool and fresh sensation.

### Example 10: Soy-containing Compositions Can Reduce Acne Lesions.

A clinical study was conducted in order to study the effect of applying a soy-containing composition twice per day for forty-five days upon subjects having mild acne (i.e., having greater than 10 non-inflammatory and inflammatory lesions at the start of the study) and upon subjects having no acne. An evaluation of acne lesion counts, an assessment of erythema and a photographic assessment were made. Soy Essence23 containing 0.005% isoflavone was applied to both groups of subjects.

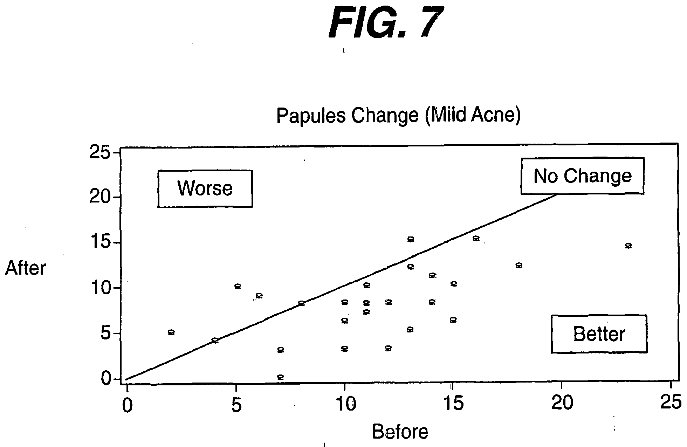

In mild acne subjects, there was a highly significant decrease from baseline in the number of inflammatory papules (p=0.001), a 41.9% decrease. There was also a directional trend toward a decrease in erythema (p=0.063) from baseline. In no acne subjects, there was no significant increase in comedones, papules or pustules. This decreased number of papules is illustrated graphically in Figure 7.

### Example 11: Soymilk-containing Compositions of This Invention Contain Intact STI

The soymilk-containing compositions of this invention are non-denatured, and have protease-inhibitory activity. These compositions are unique because they contain intact porteains, and in particular intact STI, a serine protease inhibitor. To demonstrate this uniqueness, equal volumes of Essence 23, described above in Example 3, and of a commercially available soy-based product (Helena Rubinstein's FutureWhite Essence, claiming "soja proteins" in their ingredients), were compared for their STI content using Western blos, according to Bonifacino J.S. (ed.) (1999). Immunofluoresence Staining. In Current Protocols in Cell Biology on CD-ROM. John Wiley & Sons, Inc. (Teton Data Systems, Jackson, WY). Samples were lysed in RIPA buffer [1% nonidet-P40, 0.5% sodium deoxycholate, 0.1% sodium docedyl sulfate, and Complete™ protease inhibitors (Boehringer Mannheim, Indianapolis, IN)] in PBS. RIPA lysates (10 µg per lane) were electrophoresed in 10% SDS-PAGE gels and proteins were analyzed by enhanced chemiluminescence (ECL) Western blotting (Amersham, Arlington Heights, IL). Antibodies to STI were from Chemicon and were used at a 1:500 dilution.

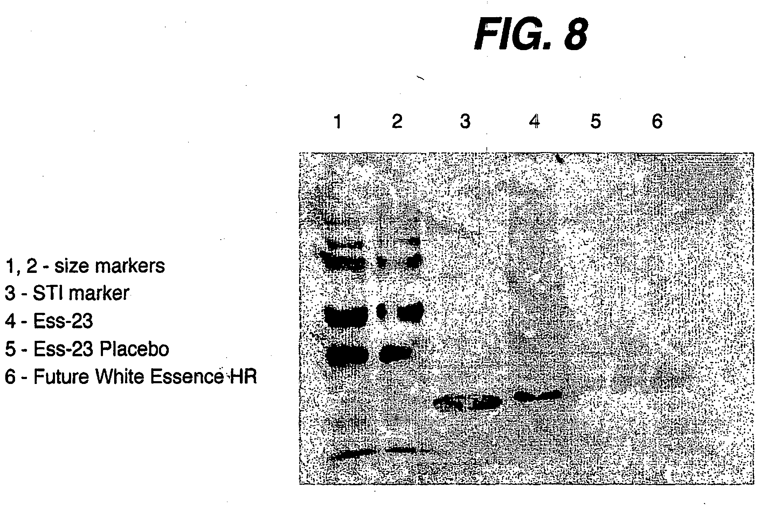

As shown in Figure 8, our soy formulation Essence 23 contains intact STI, which is equal in size to an STI marker (which is run in a parallel lane). In contrast, neither the placebo of Essence 23, nor the commercial soy-based product contains intact STI.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A cosmetic method for increasing skin firming and skin elasticity and reducing signs of skin aging comprising applying a skin composition consisting essentially of a non-denatured soy milk having protease inhibitory activity a stabilising system, said stabilising system consisting of an antioxidant, a chelating agent and a preservative; and a thickening agent, to the skin.

2. Use of soy product comprising a non-denatured soy milk having protease inhibitory activity and a stabilising system, said stabilising system comprising an antioxidant, a chelating agent or preservative, in the manufacture of a composition for the prevention or treatment of sun-induced skin damage or acne.

3. A soy product comprising a non-denatured soy milk having protease inhibitory activity and a stabilising system, comprising an anti-oxidant, a chelating agent or a preservative for use in the prevention or treatment of sun-induced skin damage or acne.

4. The method of claim 1, use of claim 2 or product of claim 3, wherein the composition contains no more than about 0.1% of a surfactant.

5. The use of claim 2 or product of claim 3, wherein the composition comprises an antioxidant.

6. The method of claim 1, or use or product of claim 5, wherein said antioxidant is BHT.

7. The use or product of any preceding claim, wherein said composition comprises a chelating agent.

8. The method of claim 1, or use or product of claim 7, wherein said chelating agent is EDTA or a derivative thereof.

9. The use or product of any preceding claim, wherein said composition comprises a preservative.

10. The method of claim 1, or use or product of claim 9, wherein said preservative is a paraben.

11. The method, use or product of any preceding claim, wherein said stabilizing system comprises BHT, EDTA or a derivative thereof and a paraben.

12. The use or product of any preceding claim, wherein the composition further comprises a thickening agent.

13. The use or product of any of claims 1 to 11, wherein the composition consists essentially of said non-denatured soy milk and said stabilizing system.

## Patentansprüche

1. Kosmetisches Verfahren zum Erhöhen der Hautstraffung und Hautelastizität und Verringerung der Anzeichen von Hautalterung, bei dem man eine Hautzusammensetzung, bestehend im Wesentlichen aus einer nichtdenaturierten Sojamilch mit Proteasehemmaktivität; einem Stabilisierungssystem, wobei das Stabilisierungssystem aus einem Antioxidationsmittel, einem Chelatisierungsmittel und einem Konservierungsmittel besteht; und einem Verdickungsmittel, topisch auf die Haut aufträgt.

2. Verwendung eines Sojaprodukts, umfassend eine nichtdenaturierte Sojamilch mit Proteasehemmaktivität und ein Stabilisierungssystem, wobei das Stabilisierungssystem ein Antioxidationsmittel, ein Chelatisierungsmittel oder ein Konservierungsmittel umfasst, in der Herstellung einer Zusammensetzung für die Vorbeugung oder Behandlung von sonneninduzierter Hautschädigung oder Akne.

3. Sojaprodukt, umfassend eine nichtdenaturierte Sojamilch mit Proteasehemmaktivität und ein Stabilisierungssystem, umfassend ein Antioxidationsmittel, ein Chelatisierungsmittel oder ein Konservierungsmittel, zur Verwendung in der Vorbeugung oder Behandlung von sonneninduzierter Hautschädigung oder Akne.

4. Verfahren nach Anspruch 1, Verwendung nach Anspruch 2 oder Produkt nach Anspruch 3, wobei die Zusammensetzung maximal ungefähr 0,1% eines Tensids enthält.

5. Verwendung nach Anspruch 2 oder Produkt nach Anspruch 3, wobei die Zusammensetzung ein Antioxidationsmittel umfasst.

6. Verfahren nach Anspruch 1, oder Verwendung oder Produkt nach Anspruch 5, wobei es sich bei dem Antioxidationsmittel um BHT handelt.

7. Verwendung oder Produkt nach einem der vorhergehenden Ansprüche, wobei die Zusammensetzung ein Chelatisierungsmittel umfasst.

8. Verfahren nach Anspruch 1, oder Verwendung oder Produkt nach Anspruch 7, wobei es sich bei dem Chelatisierungsmittel um EDTA oder ein Derivat davon handelt.

9. Verwendung oder Produkt nach einem der vorhergehenden Ansprüche, wobei die Zusammensetzung ein Konservierungsmittel umfasst.

10. Verfahren nach Anspruch 1, oder Verwendung oder Produkt nach Anspruch 9, wobei es sich bei dem Konservierungsmittel um ein Paraben handelt.

11. Verfahren, Verwendung oder Produkt nach einem der vorhergehenden Ansprüche, wobei das Stabilisierungssystem BHT, EDTA oder ein Derivat davon und ein Paraben umfasst.

12. Verwendung oder Produkt nach einem der vorhergehenden Ansprüche, wobei die Zusammensetzung weiterhin ein Verdickungsmittel umfasst.

13. Verwendung oder Produkt nach einem der Ansprüche 1 bis 11, wobei die Zusammensetzung im Wesentlichen aus der nichtdenaturierten Sojamilch und dem Stabilisierungssystem besteht.

## Revendications

1. Méthode cosmétique pour augmenter la fermeté de la peau et l'élasticité de la peau et pour réduire les signes de vieillissement cutané, comprenant l'application sur la peau par voie topique d'une composition cutanée constituée essentiellement d'un lait de soja non dénaturé ayant une activité inhibitrice de protéase ; un système stabilisant, ledit système stabilisant étant constitué d'un antioxydant, un agent chélateur et un conservateur ; et un agent épaissisant.

2. Utilisation d'un produit du soja comprenant un lait de soja non dénaturé ayant une activité inhibitrice de protéase et un système stabilisant, ledit système stabilisant comprenant un antioxydant, un agent chélateur ou un conservateur, dans la fabrication d'une composition destinée à la prévention ou au traitement de l'endommagement de la peau induit par le soleil ou de l'acné.

3. Produit du soja comprenant un lait de soja non dénaturé ayant une activité inhibitrice de protéase et un système stabilisant, comprenant un antioxydant, un agent chélateur ou un conservateur, destiné à être utilisé dans la prévention ou le traitement de l'endommagement de la peau induit par le soleil ou de l'acné.

4. Méthode selon la revendication 1, utilisation selon la revendication 2 ou produit selon la revendication 3, la composition contenant un tensioactif dans une quantité inférieure ou égale à 0,1%.

5. Utilisation selon la revendication 2 ou produit selon la revendication 3, la composition comprenant un antioxydant.

6. Méthode selon la revendication 1, ou utilisation ou produit selon la revendication 5, ledit antioxydant étant le BHT.

7. Utilisation ou produit selon l'une quelconque des revendications précédentes, ladite composition comprenant un agent chélateur.

8. Méthode selon la revendication 1, ou utilisation ou produit selon la revendication 7, ledit agent chélateur étant l'EDTA ou un dérivé de celui-ci.

9. Utilisation ou produit selon l'une quelconque des revendications précédentes, ladite composition comprenant un conservateur.

10. Méthode selon la revendication 1, ou utilisation ou produit selon la revendication 9, ledit conservateur étant un paraben.

11. Méthode, utilisation ou produit selon l'une quelconque des revendications précédentes, ledit système stabilisant comprenant le BHT, l'EDTA ou un dérivé de celui-ci et un paraben.

12. Utilisation ou produit selon l'une quelconque des revendications précédentes, la composition comprenant en outre un agent épaississant.

13. Utilisation ou produit selon l'une quelconque des revendications 1 à 11, la composition étant constituée essentiellement dudit lait de soja non dénaturé et dudit système stabilisant.
